# EUROPEAN PATENT SPECIFICATION

(11) **EP 4 551 912 B1**
(45) Date of publication and mention of the grant of the patent: **07.10.2026**
(21) Application number: 23736089.6
(22) Date of filing: 28.06.2023
(51) Int. Cl.: G01J 3/10, G01N 21/65, G01J 3/02, G01J 3/44, A61B 5/00

(54) **SENSOR MODULE FOR RAMAN SPECTROSCOPY AND ELECTRONIC DEVICE**
SENSORMODUL FÜR RAMAN-SPEKTROSKOPIE UND ELEKTRONISCHE VORRICHTUNG
MODULE DE CAPTEUR POUR LA SPECTROSCOPIE RAMAN ET DISPOSITIF ÉLECTRONIQUE

(30) Priority: 08.07.2022 DE 102022117049
(43) Date of publication of application: 14.05.2025
(73) Proprietor: ams International AG, 8645 Jona (CH)
(72) Inventor: VERDOOLD, Remco, 5661 AC Geldrop (NL); OELWEINER, Eva-Maria, 8042 Graz (AT); HASSLER, Andreas Philipp, 9020 Klagenfurt am Wörthersee (AT); HÖPPLER, Thomas, 07745 Jena (DE)
(74) Representative: Epping - Hermann - Fischer
(86) International application number: PCT/EP2023/067686
(87) International publication number: WO 2024/008536

(56) References cited:
- US-A1- 2019 195 688
- US-A1- 2021 010 865

## Description

This disclosure relates to a sensor module for Raman spectroscopy, an electronic device and a method of conducting Raman spectroscopy.

Documents US 2019/195688 A1 and US 2021/010865 A1 disclose sensor modules.

An object to be achieved is to provide a sensor module for electronic devices that overcomes limitations and provides compact means to conduct Raman spectroscopy in handheld devices. A further object is to provide an electronic device comprising such a sensor module and a method of conducting Raman spectroscopy.

These objectives are achieved with the subject-matter of the independent claims. Further developments and embodiments are described in dependent claims.

In the human body, and most vertebrates, water is used for most of the transportation of supplies and waste products. Additionally, water is used to carefully maintain a constant temperature in the body, using the advantage of the large heat capacity of water. However, a perfect balance, or hydration level, is required for optimal and most efficient performance. A small excess or dehydration does not immediately cause a problem because the body has a large number of redundant systems to counteract problems. However, when over-hydration or dehydration reaches the limit of controllable levels, it becomes dangerous and ultimately can lead to death. Under normal daily circumstances body hydration levels stay well within the save levels. But for those who perform exceptionally well (e.g., athletes or firefighters) or are no longer able to regulate their own hydration level (e.g., the elderly or dialysis patients), measuring hydration levels can become a challenge.

Most of the time body hydration is measured subjectively by performing a skin test. For example, the skin between thumb and index finger is pulled up and released, the time and discoloration could indicate body hydration. However, there are numerous factors influencing the outcome of this test, e.g. skin composition, age and the person performing the test. Alternatively, urine color, with volume, can be measured but vitamin uptake as well as minerals in a diet influences this as well. A third common method is weight measurement, sometimes assisted by body impedance, but as expected, also this measurement is exposed to numerous external factors other than hydration.

One method that is somewhat less subjective is body composition monitoring (e.g., Fresenius body composition monitor). This instrument measures the resistance differences between high and low frequency current. High frequency current is able to easily pass through intra and extra cellular media where low frequency can only pass through extra cellular media. This can provide a more consistent personalized body hydration level and is often used for people in need of dialysis.

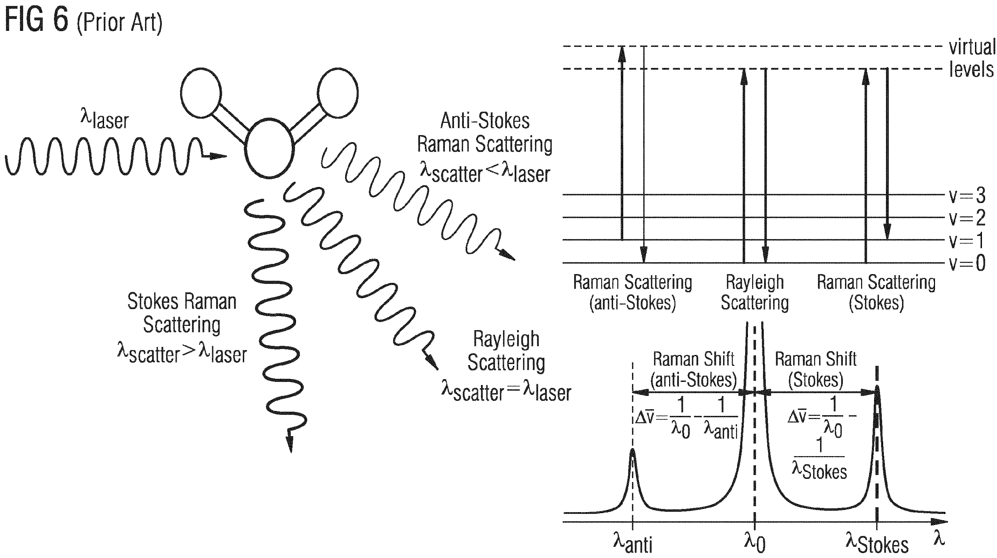

Raman spectroscopy is a technique that utilizes monochromatic light (e.g., from a laser) to determine vibrational modes of a molecule. Figure 6 shows an illustration of the Raman Effect. Briefly, laser light (i.e., photons) with a known and fixed wavelength *λₗₐₛₑᵣ* (or photon energy) is emitted and strikes a molecule. Due to energy interaction, the molecule can be excited to a higher virtual energy level state. After relaxing back from this state the photon is scattered (emission) with wavelength *λ_{scatter}*. As illustrated in the energy level scheme on the upper right corner of the drawing, the scattering is considered as Rayleigh type when the energy levels of the laser emitted photon and the scattered photon are equal, i.e. *λ_{scatter}* = *λₗₐₛₑᵣ.* Rayleigh scattering is the most observed type of scattering from an illuminated sample. The molecules relax back to their vibrational ground state v = 0. However, a smaller fraction of the scattered light will have a different photon energy than the emitted photon energy. This type of inelastic scattering is Raman scattering, where a Stokes shift results in a higher wavelength number and anti-Stokes in a lower wavelength number.

Stokes shifts and anti-Stokes shifts involve higher vibrational states v > 0. Thus, the vibration of molecules within a sample will result in a Raman (Stokes and Anti-Stokes) scattering with intensities spread over multiple wavenumbers, which is visualized in the spectrum plot on the bottom left corner of the drawing. As common in Raman spectroscopy, the emitted photon energy is not expressed in wavelength (seen by the spectrometer instrument), but in wavenumbers cm⁻¹, which is the energy shift with respect to the excitation wavelength. Additionally, it is this wavenumber shift, from the excitation wavelength, that is used (Raman shift) for analysis of data. The Raman shift *υ̅* derives from $\overline{υ} = \frac{1}{{λ}_{laser}} - \frac{1}{{λ}_{emission}}$ wherein *λₑₘᵢₛₛᵢₒₙ* denotes the emitted Raman peak wavelength, e.g. on a spectrometer.

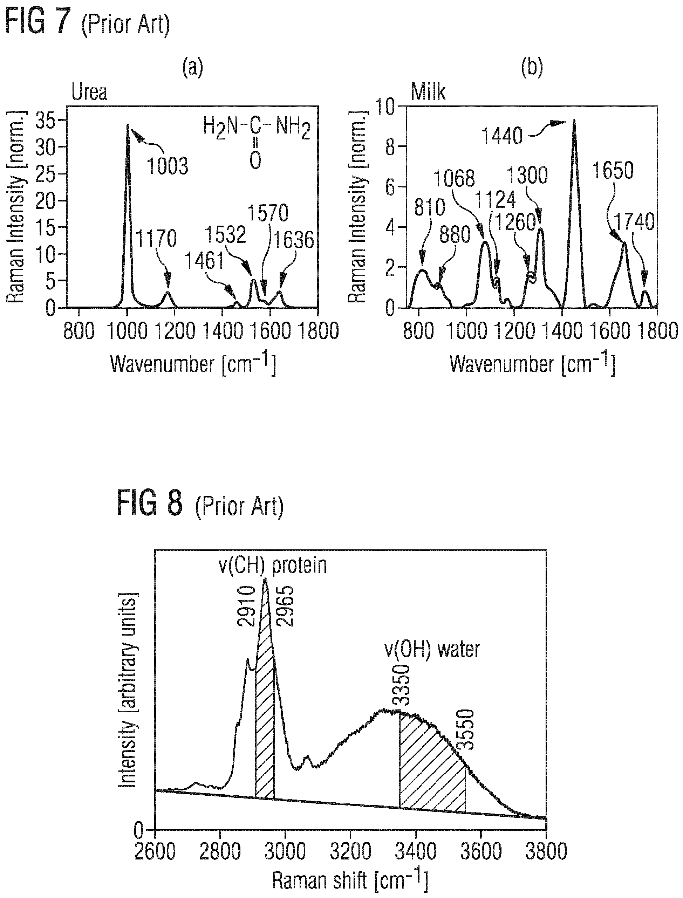

To date Raman spectrometers often rely on research-grade instrumentation and benchtop systems. Normally, a tuned spectrometer (e.g., a grating with line-pixel sensor) is connected to a lens that receives the Raman scattered light. In the optical path, an optical notch filter is placed that prevents Rayleigh scattering of the laser excitation wavelength *λₗₐₛₑᵣ* from interfering with the sensor signal or alternatively, a band pass filter is used to stop the excitation light and pass the signal wavelengths of the Raman spectrum. Two very typical Raman spectra are depicted in Figure 7 with graphs of urea on the left and milk on the right. Urea shows a very strong peak at 1003 cm⁻¹ and milk is a more complex medium with more equal intensity peaks. Visible are peak positions that correspond to present atoms and their bond types. With this molecular fingerprint, it is possible to determine the relative content in complex media.

The capability of Raman scattering of measuring at the molecular selectivity enables to measure levels of individual molecule types in complex media. This enables also to measure the water content in the skin. Figure 8 shows a typical Raman spectrum of the skin. Depicted is an in-vivo Raman spectrum of the stratum corneum obtained from a human arm. The water protein ratio can be calculated as the ratio between the integrated signal intensities of the gray areas. Experimental conditions include a signal collection time of 3 s, laser power at 100 mW, and excitation wavelength of 720 nm.

There are two main peaks in the Raman spectrum, one for solids of cells, like proteins and lipids and the second for water content of the cells and extracellular media. The solid content will remain mostly stable at various levels of water content. The spectral graph illustrates the variation in hydration level, by change in peak intensity for the water Raman band. For the actual measurement of water content in skin it is not required to have absolute Raman spectral information but a ratio of the two highlighted bands would be sufficient. This enables using only two spectral filters. One that gives the signal intensity for proteins and lipids, and a second filter that gives the signal intensity for water. The ratio between those two numbers is an indication for hydration. It does require a normality calibration to obtain a normal hydration level ratio. However, this can also be obtained from trend information during multi day measurements.

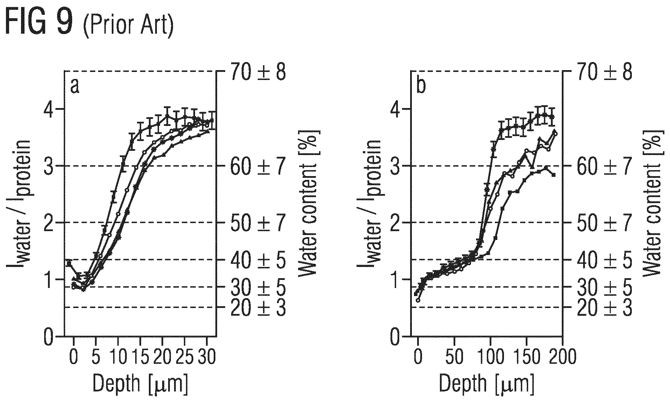

The level of hydration or water in the skin varies with the depth from the skin surface (see Thesis - Peter Caspers, in-vivo skin characterization by confocal Raman micro spectroscopy, 2003, university of Rotterdam, isbn 978-90-6734-366-4 and Peter J. Caspers, Gerald W. Lucassen, Elizabeth A. Carter, Hajo A. Bruining, and Gerwin J. Puppels, (2001), In Vivo Confocal Raman Microspectroscopy of the Skin: Noninvasive Determination of Molecular Concentration Profiles, 2001, VOL. 116, NO. 3 MARCH 2001, IN VIVO RAMAN SPECTROSCOPY OF SKIN by The Society for Investigative Dermatology, Inc.). At a depth of 200 µm from the skin surface the hydration level is approximately 65% as illustrated in Figure 9. Figure 9 shows in-vivo water concentration profiles of the stratum corneum, as found in the citations above. The graph on the left shows four water concentration profiles, calculated from Raman measurements on the volar aspect of the forearm. Different symbols were used for profiles obtained for different measurement locations. The graph on the right shows four water concentration profiles based on Raman measurements on the thenar. Different symbols demarcate different measurement locations. The left hand ordinate is the ratio between the Raman signal intensities of water and protein. The right hand ordinate represents the calculated absolute water content in mass-% (grams of water per 100 g of wet tissue). Note that this is a non-linear scale.

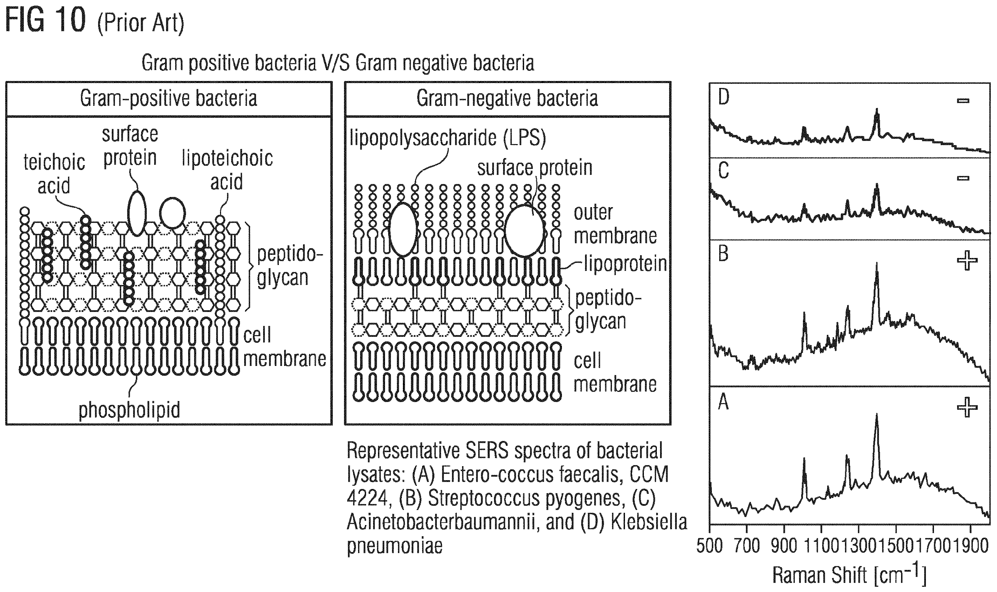

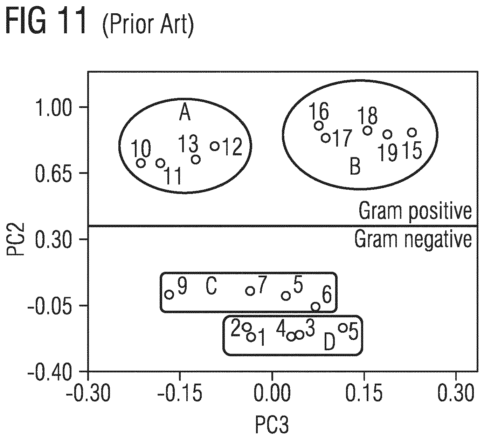

In another application, Raman spectroscopy can be used to conduct measurements of pathogens such as bacteria. Every organism will have different ratios of chemicals and molecules present in their body. The simpler the organism the less complex the Raman spectrum would look like. For example, in bacteria there are various classifications. At first there is GRAM positive or negative, this is an indicator for the construction of the cell membrane as shown in Figure 10. Here, it is possible to see that different molecules are present and that this will influence the Raman response from these types of organisms. Figure 10 shows Gram positive vs Gram negative on the left. On the right Raman SERS spectrum of two Gram positive and two Gram negative bacteria. Next to the membrane structure of bacteria their content like proteins and amino acids will add to the complexity of the Raman spectrum. Using a Cluster analysis one can separate the complexity between samples as shown in Figure 11. Figure shows 11 shows a cluster analysis of Gram positive and Gram negative bacteria.

Lately there has been a tremendous advancement in the field of photonics. Thanks to photonics Raman spectroscopy has become much more accessible to users in all fields and provides more objective means for various aspects of applications, such as Biosensing. Footprints of complete spectrometers can be further miniaturized with the help of ever more compact integrated optics, optoelectronics and laser sources. However, Raman spectrometers still remain research-grade instrumentation and benchtop systems, despite the growing need for mobile devices such as mature spectrometers in Smart Watches, medical and point-of-care or other handheld devices. Biosensing applications to detect skin constituents like urea, lactate and interstitial fluid or blood glucose are only one example, measurements of pathogens (e.g. bacteria) another. Compact handheld devices are expected to have a huge impact on most potential spectrometer applications, e.g. material analysis, environmental analysis, biosensors, Smart Health, Medsumer, point-of-care, medical etc.

The following relates to an improved concept in the field of Raman spectroscopy. One aspect relates to a sensor module for Raman spectroscopy, i.e. a miniaturized measurement system to conduct Raman spectroscopy. Possible applications comprise a fingerprinting measurement system of bacteria or viruses. This system can be used for many other measurement applications as well, such as ripeness of fruits, quality of foods, raw material safety etc. Another application includes skin hydration, e.g. by measuring the ratio between solids and water content of the skin using a single wavelength band filter to detect the Raman peaks.

Further aspects relate to a combination of a spectral sensor with a number of diodes, integrated in an ASIC, or a number of diodes with spectral filters readout by an ASIC, in combination with only one or a few narrowband light emitter(s) and a miniature housing, all fitting in a volume of smaller than 2 cm³. Potentially, the light emitter can be modulated, which modulation frequency may be seen back in the intensity modulation of the detected Raman signal. Additionally, multiple excitation sources can be used to spread the power density over the skin and stay below safety limits for skin illumination. Additionally, different lenses can have different focus depths to create a depth profile of the skin, with respect to water or any other Raman active material of the skin or tissue.

In accordance with the claimed invention, a sensor module for Raman spectroscopy comprises a sensor package. The sensor package encloses an application specific integrated circuit, or ASIC for short, a light emitter arrangement, a light detector arrangement and a filter arrangement.

The light emitter arrangement, e.g. a semiconductor light emitter arrangement, is electrically connected to the ASIC and operable to emit light with multiple excitation wavelengths to excite Raman scattering in an external probe to be placed outside of the sensor module. The light detector arrangement is operable to generate sensor signals from incident light emitted back from the external probe due to the Raman scattering. The filter arrangement is operable to filter the incident light according to a target passband. The ASIC is operable to drive the light detector arrangement at the excitation wavelengths to shift a Raman spectral band of the external probe into the passband of the filter arrangement.

The shifting of Raman spectral band allows to probe sections of a Raman spectrum with just a single filter or a set of filters to extend the range of sections which can be probed. This way, the sensor module eliminates the use of a high resolution spectrometer and complex optics and replaces this by a number of excitation wavelengths and set of well-defined passband filters. This greatly simplifies the measurement system and reduces cost prices from tens of thousands to tens of dollars. As a consequence, Raman spectroscopy may be conducted in handheld devices.

In at least one embodiment, the light emitter arrangement comprises a single light emitter operable to emit light with multiple emission lines according to the multiple excitation wavelengths. A single light emitter may suffice to provide multiple excitation wavelengths, which renders the sensor module more compact. Said light emitter may emit discrete multiple excitation wavelengths all at once, so that individual lines may need to be filtered out.

In at least one embodiment, the light emitter arrangement comprises a single tuneable light emitter operable to emit light with tuneable emission lines according to the multiple excitation wavelengths. A single light emitter may suffice to provide multiple excitation wavelengths, which renders the sensor module more compact. Said tuneable light emitter may emit a single discrete excitation wavelength at a time and be tuned to emit another. This way there may be no need to filter out individual lines.

In at least one embodiment, the light emitter arrangement comprises an array of light emitters with different emission lines. The light emitters of the array are operable to emit light with at least one emission line according to the multiple excitation wavelengths. An array of light emitters may require more space but could be manufactured more easily by means of CMOS technology, for example. Instead of tuning, multiple excitation wavelengths may readily be available, e.g. under control of the ASIC.

In at least one embodiment, the light emitter arrangement comprises at least one of a laser diode or a laser surface emitter, e.g. a VCSEL, as light emitter.

In at least one embodiment, the light detector arrangement comprises a single light detector and/or a single semiconductor light detector. The filter arrangement comprises a filter which is arranged in front of the light detector. A single light detector may suffice as spectral features of the probe are shifted into the passband of the filter or passbands of filters due to the multiple excitation wavelengths, which renders the sensor module more compact.

In at least one embodiment, the light detector arrangement comprises an array of light detectors and/or an array of semiconductor light detectors. The filter arrangement comprises multiple filters arranged in front of the light detectors, respectively. The array may be arranged with a single filter to increase signal-to-noise ratio, for example. Instead there may be a dedicated filter in front of respective light detectors in order to be able to probe more sections or spectral features of an external probe. Furthermore, using an array also facilitates use of the sensor module for more external probes, as the target passbands can be adjusted to cover more spectral features.

In at least one embodiment, the filter in front of the single light detector comprises a broad passband, or broadband-filter for short. Alternatively, multiple filters arranged in front of the light detectors comprise narrow passbands, or narrowband-filters for short. Bandwidth may account for the expected width of spectral features, i.e. peaks or bands, in the desired Raman spectra.

In at least one embodiment, the narrowband-filters have non-overlapping passbands with discrete center wavelengths. Non-overlap may further spread the spectral range that can be probed.

In at least one embodiment, the light detector arrangement comprises at least one of a photon counter, e.g. a single photon avalanche diode, or SPAD, an avalanche photo diode, or APD, a silicon photomultiplier, or SiPM, a photodiode or a charge coupled device, or CCD, or a MEMS photo multiplier, or PM, as light detector.

The ASIC further comprises a modulator and a lock-in amplifier. The modulator is operable to provide an AC drive signal with a modulation frequency and to provide a reference signal associated with the AC drive signal. Furthermore, the lock-in amplifier is operable to receive the sensor signals and to receive the reference signal from the modulator, and to perform phase-locked detection of the modulation frequency in the sensor signals to determine a phase and an amplitude from the sensor signals using the reference signal. Modulated excitation enables to do phase-locked detection of the modulation frequency in the Raman sensor signal, which further improves the sensitivity and accuracy of the detection.

In at least one embodiment, the sensor package further encloses a lens arrangement. The lens arrangement is arranged to direct the emitted light to the external probe to excite Raman scattering. In addition, or alternatively, the lens arrangement is arranged to direct the incident light to the light detector arrangement. The lens arrangement may increase signal-to-noise ratio by way of focusing in both excitation and detection. Furthermore, the lens arrangement may provide different focal lengths in order to excite the external probe with different penetration depths.

In at least one embodiment, the lens arrangement is further operable to direct the emitted light to the external probe under an angle different from normal incidence so as to provide angled illumination with an angled illumination source which helps to reduce the impact of auto fluorescence from the target.

In at least one embodiment, an electronic device comprises a sensor module for Raman spectroscopy according to one of the aforementioned aspects. The electronic device comprises a host system and the sensor module is embedded in and electrically connected to the host system. The host system comprises one of a mobile device, smartphone, handheld computer, Smart Watch, medical device, point-of-care device.

Furthermore, a method of conducting Raman spectroscopy is suggested. The method is conducted using a sensor module comprising a sensor package which encloses an application specific integrated circuit, or ASIC, a light emitter arrangement electrically connected to the ASIC, a light detector arrangement and a filter arrangement. The method comprises the step of emitting light with multiple excitation wavelengths to excite Raman scattering in an external probe to be placed outside of the sensor module. Another steps involves generating sensor signals from incident light emitted back from the external probe due to the Raman scattering. Another steps involves filtering the incident light according to a target passband. Another steps involves, using the ASIC, driving the light detector arrangement at the excitation wavelengths to shift a Raman spectral band of the external probe into the passband of the filter arrangement.

Further embodiments of the method become apparent to the skilled reader from the aforementioned embodiments of the sensor module and of the electronic device, and vice-versa.

The proposed concept may have one or more of the following advantages:
- less components needed,
- offers more integrated solution, e.g. less parasitics enables to better measure the weak Raman signals and application of modulated excitation may enable to do phase-locked detection of the modulation frequency in the Raman signal, which further improves the sensitivity and accuracy of the detection,
- allows to implement true point-of-care device. Currently, spectroscopy measurements are almost exclusively performed at the clinical laboratory,
- possibility for point-of-care measurement with disposable components,
- performance, low cost, low COGS,
- possibility to implement in wearable sensor host systems,
- potential to have absolute measurements of body hydration on the measurement location,
- an excitation from multiple sources may decrease the illumination density at the skin entrance, well within safety limits, while keeping sufficient intensity at the focal point for the Raman Signal generation and different lenses on each excitation source can have variable focus depth, through which a depth curve can be measured.
- sensor module can be as small as volume <2 cm³

### BRIEF DESCRIPTION OF THE DRAWINGS

The following description of figures may further illustrate and explain aspects of the sensor module for Raman spectroscopy, electronic device and the method of conducting Raman spectroscopy. Components and parts of the sensor module that are functionally identical or have an identical effect are denoted by identical reference symbols. Identical or effectively identical components and parts might be described only with respect to the figures where they occur first. Their description is not necessarily repeated in successive figures.

In the figures:
- Figure 1: shows a first exemplary embodiment of a sensor module for Raman spectroscopy,
- Figure 2: shows Raman spectra with an example set of spectral bands and excitation wavelengths for the first exemplary embodiment,
- Figure 3: shows a second exemplary embodiment of a sensor module for Raman spectroscopy,
- Figure 4: shows a third exemplary embodiment of a sensor module for Raman spectroscopy,
- Figure 5: shows Raman spectra with an example set of spectral bands and excitation wavelengths for the third exemplary embodiment,
- Figure 6: shows an illustration of the Raman Effect,
- Figure 7: shows Raman spectra of urea and milk,
- Figure 8: shows an in-vivo Raman spectrum of the stratum corneum obtained from a human arm,
- Figure 9: shows in-vivo water concentration profiles of the stratum corneum,
- Figure 10: shows an illustration of Gram positive vs Gram negative bacteria, and
- Figure 11: shows a cluster analysis of Gram positive and Gram negative bacteria.

### DETAILED DESCRIPTION

In the following several exemplary embodiments of a sensor module for Raman spectroscopy are presented. The sensor module comprises a sensor package which encloses the components of the module. For example, the sensor package comprises a molded housing (not shown) to mount or place the components into. The sensor module encloses components including an application specific integrated circuit 10, or ASIC for short, a semiconductor light emitter arrangement 20, a semiconductor light detector arrangement 30 and a filter arrangement 40. In the examples discussed below a lens arrangement is also arranged into the sensor package. Alternatively, a non-semiconductor light emitter arrangement and/or light detector arrangement can be implemented.

For example, the molded housing comprises a hollow molded body which is mounted on and connected to the ASIC 10, e.g. by means of a carrier. Furthermore, the semiconductor light emitter arrangement 20 and the semiconductor light detector arrangement 30 are placed behind respective apertures to emit light out of the sensor module and receive incident light. The housing can be arranged with chambers, one chamber for the semiconductor light emitter arrangement and another chamber for the semiconductor light detector arrangement. The semiconductor light emitter arrangement and the semiconductor light detector arrangement can be optically isolated by means of a light barrier, e.g. a wall in the housing separating the chambers.

The semiconductor light detector arrangement can be integrated into the ASIC 10, or, together with the ASIC, form an integrated circuit, such as a CMOS integrated circuit, mounted on a common substrate or carrier. The semiconductor light emitter arrangement 20 can either be integrated into the ASIC or the integrated circuit or be electrically connected to the integrated circuit or ASIC as external components.

The semiconductor light emitter arrangement 20 comprises one or more light emitters 21, such as semiconductor laser diodes and/or resonant cavity light emitting devices. These devices feature coherent emission to generate light at various excitation wavelengths. A resonant cavity light emitting device can be considered a semiconductor device which is operable to emit coherent light based on a resonance process. In this process, the resonant cavity light emitting device may directly convert electrical energy into light, e.g. when pumped directly with an electrical current to create amplified spontaneous emission. However, instead of producing stimulated emission only spontaneous emission may result, e.g. spontaneous emission perpendicular to a surface of the semiconductor is amplified.

One example relates to vertical cavity surface emitting laser, VCSEL, diodes. VCSELs are an example of a resonant cavity light emitting device and feature a beam emission that is perpendicular to a main extension plane of a top surface of the VCSEL. The VCSEL diode can be formed from semiconductor layers on a substrate, wherein the semiconductor layers comprise distributed Bragg reflectors enclosing active region layers in between and thus forming a cavity. VCSELs and their principle of operation are a well-known concept and are not further detailed throughout this disclosure. For example, the VCSEL diode is configured to have an emission wavelength of 940 nm, 850 nm, or another natural wavelength. Other emission wavelengths include 660 nm, 671 nm, 680 nm, and 785 nm. However, VCSELs can also be tuneable and driven by the ASIC to emit at various wavelengths. The VCSEL diode can be configured to emit coherent laser light when forward biased, for instance.

The semiconductor light detector arrangement comprises one or more light detectors 31, such as photon counters, e.g. single photon avalanche diodes, or SPADs, avalanche photo diodes, or APDs, silicon photomultipliers, or SiPMs, semiconductor photodiodes or charge coupled devices, or CCDs, or MEMS photo multipliers, or PMs.

The light detectors 31 are complemented with the filter arrangement 40, which filters incident light according to one or more target passbands. The filter arrangement comprises one or more optical filters 41, such as interference filters or dichroic filters, Plasmon filters and/or absorption filters, or a combination thereof. The filters can be arranged in a filter layer with one or more sections dedicated to a respective light detector. The filters may also be placed on or integrated into a corresponding light detector.

The target passband of a filter 41 can be a broad passband, i.e. the filter is a broadband-filter, or narrow passbands, i.e. the filter is a narrowband-filter. Broad is considered 50 nm and larger and narrow is considered smaller than 10 nm, for example. The term "target" is used to indicate that the passband is chosen with a desired target or probe in mind. For example, a substance to be measured is known to feature a Raman spectral band in a defined spectral range when excited with a given excitation wavelength. Then the target passband can be chosen to cover (or pass) said Raman spectral band. This way the passband may cover a characteristic spectral feature of a probe and, in turn, it may suffice that only said spectral band is probed to detect the desired target, or substance.

Furthermore, the sensor package comprises a lens arrangement 50 with lenses 51, e.g. micro-lenses, placed on or integrated directly into a corresponding light detector 31. The lens arrangement directs emitted light to an external probe 60 to excite Raman scattering. Furthermore, the lens arrangement may also be used to direct the incident light to the light detector arrangement. For example, lenses can have different focal lengths to focus emitted light into different depths of the external target. Moreover, lenses may be tilted in the sensor package so as to direct the emitted light to the external probe under an angle different from normal incidence. This way, angled illumination of the external target is possible with an angled illumination source which helps to reduce the impact of auto fluorescence from the target. Furthermore, additional lenses may be provided to focus incident light onto the respective light detectors to increase signal-to-noise levels.

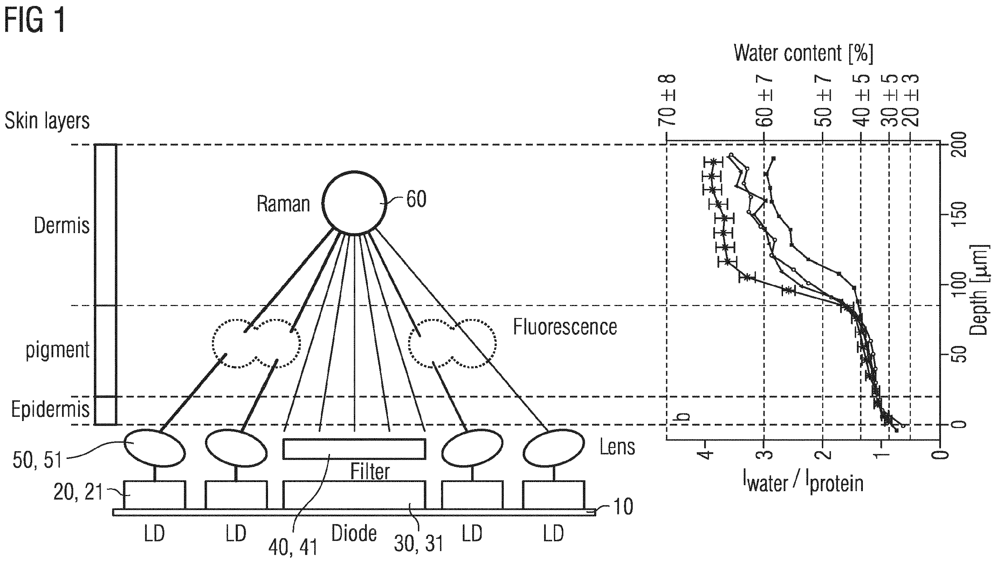

Figure 1 shows a first exemplary embodiment of a sensor module for Raman spectroscopy. In this embodiment the filter arrangement 40 comprises only a single optical filter 41, and a high performance light detector 31 forms the semiconductor light detector arrangement 30 on the sensing side. Alternatively, more than a single light detector can form the semiconductor light detector arrangement and be placed behind the same single optical filter 41 to further increase signal-to-noise ratio. On the source side the semiconductor light emitter arrangement 20 is formed by four different light emitters 21, for example, such as laser diodes, each operable to emit with a unique excitation wavelength. This greatly simplifies the measurement system. Furthermore, this embodiment employs tilted lenses which form the lens arrangement in front of the light emitters.

In operation, light is excited by the light emitters 21 and focused to a specific depths at the external target, e.g. at 80 to 500 µm into the dermis of human tissue. Other depths are possible as shown by the depth-water graph on the right hand side of the drawing. Raman emission from molecules is scattered and received by the light detector 31. Due to the filter 41 only light with wavelengths defined by the target passband pass and are incident on the light detector to generate respective sensor signals. In this example, the filter accepts energy levels for solids or water.

The sensor signals can be analyzed, e.g. by the ASIC 10 or by an external processing unit (not shown). For example, a ratio of sensor signals (or channels) collected for different excitation wavelengths can give an indication of the presence of a given spectral feature, e.g. a peak or band, of the external target 60. For example, a ratio of sensor signals related to protein and water may give an indication of body hydration. The melanin in skin will result in auto fluorescence, therefore the angle of illumination could differ from the position of reception.

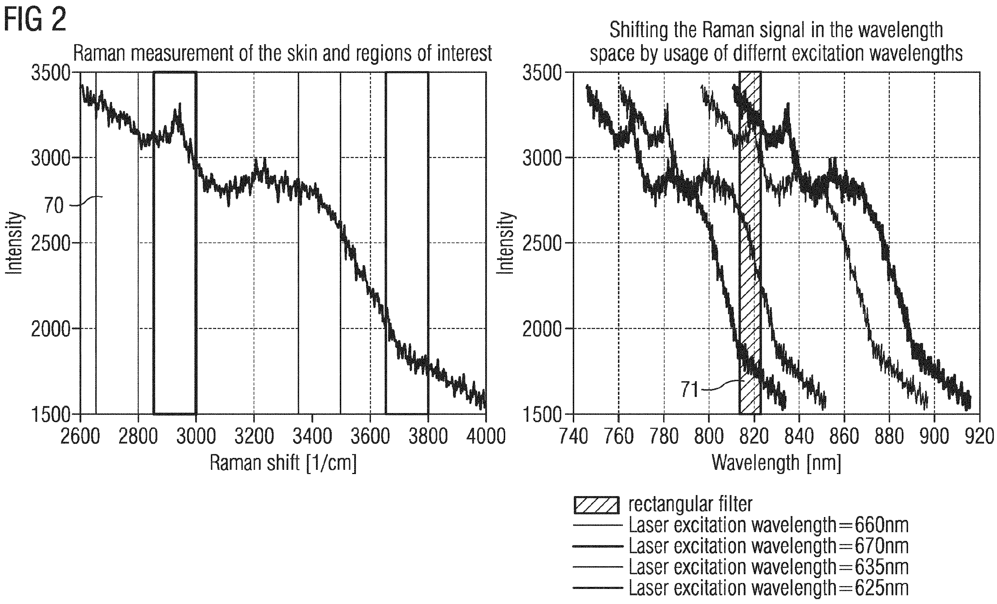

Figure 2 shows Raman spectra with an example set of spectral bands and excitation wavelengths for the first exemplary embodiment. The graph on the left shows a Raman spectrum of a human skin, intensity in arbitrary units vs. Raman shift in wavenumber cm⁻¹. The graph further indicates four spectral bands 70, or regions of interest. These regions of interest are related to the four excitation wavelengths which can be provided by the light emitters. In this example, a first excitation wavelength is 660 nm, a second excitation wavelength is 670 nm, a third excitation wavelength is 635 nm, and a fourth excitation wavelength is 625 nm.

The graph on the right represents intensity in arbitrary units vs. wavelength in nm. The rectangular area represents the target passband 71 of the optical filter 41. Furthermore, four Raman spectra are depicted, resulting from excitation with the four excitation wavelengths, respectively. As a consequence of the different excitation, the Raman spectra are shifted with respect to wavelength space. For example, a peak in the Raman spectrum is shifted into the target passband using the first excitation wavelength at 660 nm. In other words, changing the excitation wavelengths shifts different Raman spectral bands of the external probe (here human skin) into the passband of the filter. This way, the external target 60 can be probed in sections, or regions of interest.

The measurements performed with a desktop Raman spectrometer, however, involve computing or probing the complete scattering spectrum of the Raman signal. However, as the measurement discussed in Figure 1 uses different excitation wavelengths, merely part of the Raman spectrum can be probed, e.g. regions of interest, which include a desired spectral feature, e.g. a peak or band of a known specimen or substance. For example, for detecting the Raman signal peaks of solids and water, by way of shifting the spectra into a single target passband 71 a single filtered sensor could be sufficient.

It should be noted that hydration is not the only potential use case of this device. Equally for other compounds more than two specific wavelengths can be selected and detected by dedicated light detectors with optical filters 41 on top, either separated or integrated in a spectra sensor configuration, embedded in a readout ASIC 10.

An advantage of using a single spectral filter 41 over multiple filters is that the light detector(s) 31 can have a large surface area, and optimize the angle of incidence of the lenses. Also the filter fabrication step is greatly simplified and will have reduced stresses in the material because only a single filter is present on the carrier medium (e.g. glass). Additionally, the light emitters 21 can be all placed around the sensor or a single laser can be tuned to various wavelengths as will be discussed next.

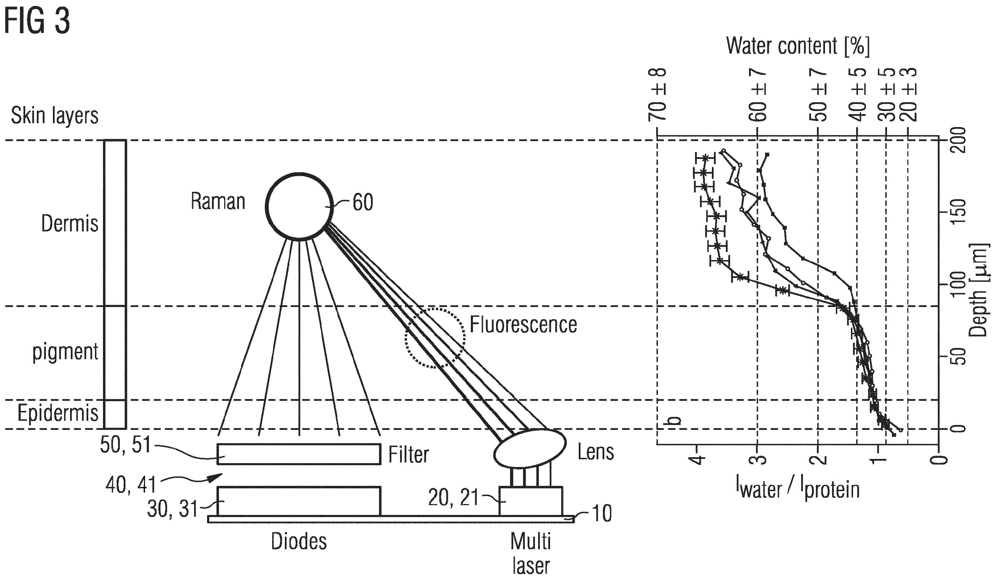

Figure 3 shows a second exemplary embodiment of a sensor module for Raman spectroscopy. This example is closely related to the one discussed in respect of Figure 1. Instead of four single light emitters 21, however, a single light emitter is used. This single light emitter is operable to emit light with four different excitation wavelengths, e.g. the first to fourth excitation wavelengths introduced above. In general, it is possible to have the excitation wavelengths realized as distinct emission lines or instead the light emitter is tuneable to provide emission lines from a continuous spectral range. This further simplifies the measurement system, e.g. in terms of space requirement and compactness. For example, semiconductor laser diodes such as VCSELs can be designed with tuneable emission.

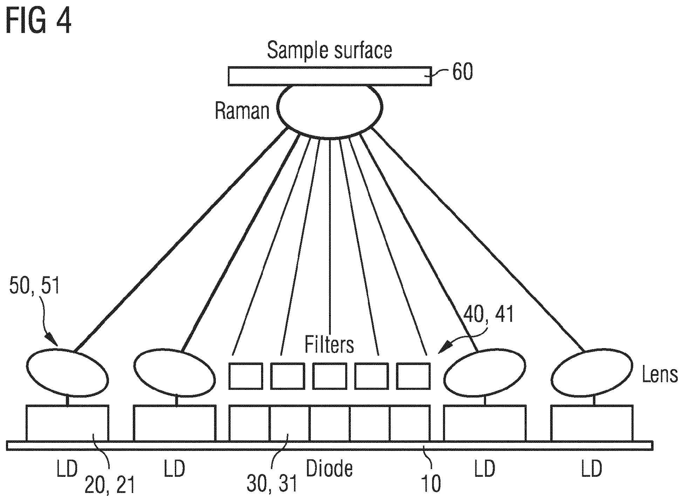

Figure 4 shows a third exemplary embodiment of a sensor module for Raman spectroscopy. In this embodiment the filter arrangement 40 comprises several optical filters 41 with different target passbands 71 (here five as an example), and an array of respective (five) light detectors 31 forms the semiconductor light detector arrangement 30 on the sensing side. On the source side the semiconductor light emitter arrangement 20 is formed by four different light emitters 21, such as laser diodes, each operable to emit with a unique excitation wavelength. This greatly simplifies the measurement system. Furthermore, this embodiment employs tilted lenses which form the lens arrangement in front of the light emitters.

Operation of the sensor module is as discussed above. A difference involves that the Raman spectra can be shifted according to the excitation wavelengths into several target passbands 71, as opposed to a single passband as discussed above. For example, using five spectral band filters with respective non-overlapping target passbands, numerous spectral features, e.g. peaks and bands of interest, can be measured. By using three excitation wavelengths it is possible to measure three Raman bands with a single filter. So in general with five filters and four light emitters a total of 20 spectral bands can be measured.

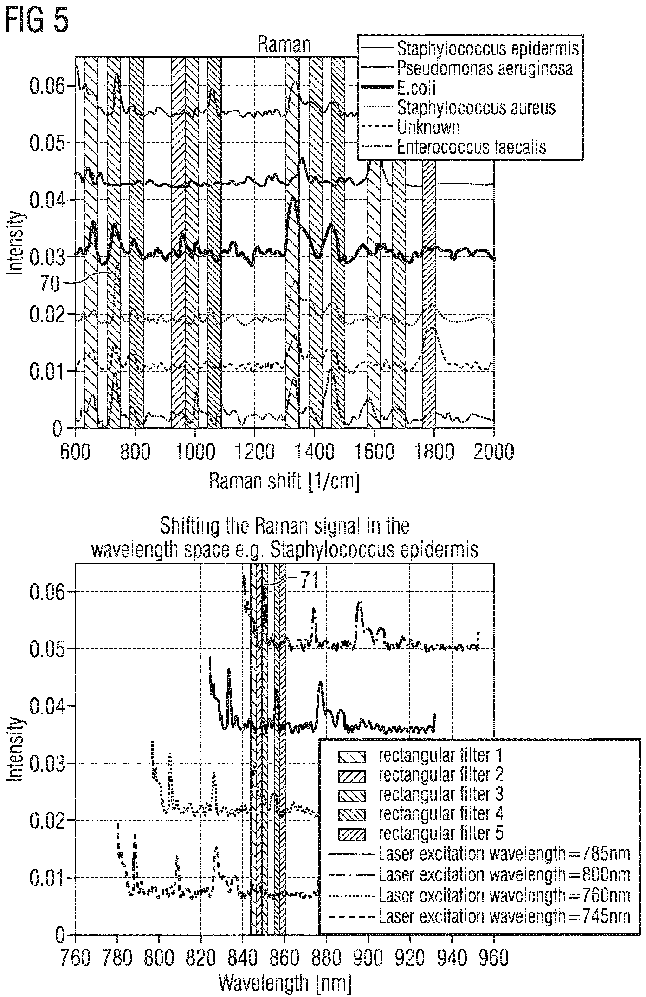

Figure 5 shows Raman spectra with an example set of spectral bands and excitation wavelengths for the third exemplary embodiment. The graph on the left shows Raman spectra of various bacteria, intensity in arbitrary units vs. Raman shift in wavenumber cm⁻¹. The graph further indicates several bands or regions of interest. These regions of interest are related to the excitation wavelengths which can be provided by the light emitters. In this example, a first excitation wavelength is 785 nm, a second excitation wavelength is 800 nm, a third excitation wavelength is 760 nm, and a fourth excitation wavelength is 745 nm.

The graph on the right represents intensity in arbitrary units vs. wavelength in nm. The rectangular areas represent the target passbands 71 of the optical filters 41. Four Raman spectra of Staphylococcus epidermis are depicted, resulting from excitation with the four different excitation wavelengths, respectively. As a consequence of the different excitation, the Raman spectra are shifted with respect to wavelength space. For example, different peaks of the Raman spectrum are shifted into the target passbands 71 using the different excitation wavelengths. In other words, changing the excitation wavelengths shifts different Raman spectral bands of the external probe 60 (here Staphylococcus epidermis) into the passbands 71 of the filters 41. This way, the external target can be probed in sections, or regions of interest.

The measurements performed with a desktop Raman spectrometer cover the complete scattering spectrum of the Raman signal. However, as the measurement discussed in Figure 4 uses different excitation wavelengths, merely parts of the Raman spectrum can be probed, e.g. regions of interest, which include desired spectral features, e.g. a peak or band of a known specimen or substance. For example, for detecting the Raman signal peaks of bacteria, by way of shifting the spectra a target passbands and/or a filtered sensor could be sufficient.

While this specification contains many specifics, these should not be construed as limitations on the scope of the invention or of what may be claimed, but rather as descriptions of features specific to particular embodiments of the invention. Certain features that are described in this specification in the context of separate embodiments can also be implemented in combination in a single embodiment.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous.

Furthermore, as used herein, the term "comprising" does not exclude other elements. In addition, as used herein, the article "a" is intended to include one or more than one component or element, and is not limited to be construed as meaning only one.

This patent application claims the priority of German patent application 102022117049.

### References

- 10: application specific integrated circuit, ASIC
- 20: light emitter arrangement
- 21: light emitter
- 30: light detector arrangement
- 31: light detector
- 40: filter arrangement
- 41: filter
- 50: lens arrangement
- 51: lens
- 60: external probe
- 70: spectral band
- 71: target passband

## Claims

1. A sensor module for Raman spectroscopy, comprising a sensor package which encloses:
- an application specific integrated circuit (10), or ASIC,
- a light emitter arrangement (20) electrically connected to the ASIC (10) and operable to emit light with multiple excitation wavelengths to excite Raman scattering in an external probe (60) to be placed outside of the sensor module,
- a light detector arrangement (30) operable to generate sensor signals from incident light emitted back from the external probe (60) due to the Raman scattering, and
- a filter arrangement (40) operable to filter the incident light according to a target passband (71); and wherein:
the ASIC (10) is operable to drive the light emitter arrangement (20) at the excitation wavelengths to shift a Raman spectral band of the external probe into the passband of the filter arrangement (40), wherein the ASIC (10) further comprises:
- a modulator operable to provide an AC drive signal with a modulation frequency and to provide a reference signal associated with the AC drive signal,
- a lock-in amplifier operable to receive the sensor signals and to receive the reference signal from the modulator, and to perform phase-locked detection of the modulation frequency in the sensor signals to determine a phase and an amplitude from the sensor signals using the reference signal.

2. The sensor module according to claim 1, wherein the light emitter arrangement (20) comprises a single light emitter (21) operable to emit light with multiple emission lines according to the multiple excitation wavelengths.

3. The sensor module according to claim 1 or 2, wherein the light emitter arrangement (20) comprises a single tuneable light emitter (21) operable to emit light with tuneable emission lines according to the multiple excitation wavelengths.

4. The sensor module according to one of claims 1 to 3, wherein
- the light emitter arrangement (20) comprises an array of light emitters (21) with different emission lines, and
- the light emitters (21) of the array are operable to emit light with at least one emission line according to the multiple excitation wavelengths.

5. The sensor module according to one of claims 1 to 4, wherein the light emitter arrangement (20) comprises at least one of a laser diode or a laser surface emitter, e.g. a VCSEL, as light emitter.

6. The sensor module according to one of claims 1 to 5, wherein
- the light detector arrangement (30) comprises a single light detector (31), and
- the filter arrangement (40) comprises a filter (41) which is arranged in front of the light detector (31).

7. The sensor module according to one of claims 1 to 5, wherein
- the light detector arrangement (30) comprises an array of light detectors (31), and
- the filter arrangement (40) comprises multiple filters (41) arranged in front of the light detectors (31), respectively.

8. The sensor module according to claim 6 or 7, wherein:
- the filter (41) in front of the single light detector (31) comprises a broad passband, or broadband-filter, or
- multiple filters arranged in front of the light detectors (31) comprise narrow passbands, or narrowband-filters.

9. The sensor module according to claim 8, wherein the narrowband-filters have non-overlapping passbands with discrete center wavelengths.

10. The sensor module according to one of claims 1 to 9, wherein the light detector arrangement (30) comprises at least one of a photon counter, e.g. a single photon avalanche diode, or SPAD, an avalanche photo diode, or APD, a silicon photomultiplier, or SiPM, a photodiode or a charge coupled device, or CCD, or a MEMS photo multiplier, or PM, as light detector (31).

11. The sensor module according to one of claims 1 to 10, wherein
- the sensor package further encloses a lens arrangement (50), and
- the lens arrangement (50) is arranged to direct the emitted light to the external probe (60) to excite Raman scattering, and/or
- the lens arrangement (50) is arranged to direct the incident light to the light detector arrangement (30).

12. The sensor module according to claim 11, wherein the lens arrangement (50) is further operable to direct the emitted light to the external probe (60) under an angle different from normal incidence so as to provide angled illumination.

13. The sensor module according to at least one of the previous claims, wherein the light detector arrangement (30) is integrated into the ASIC (10) and/or the semiconductor light emitter arrangement (20) is integrated into the ASIC (10).

14. An electronic device, comprising:
- a sensor module for Raman spectroscopy according to one of claims 1 to 13, and
- a host system comprising one of a mobile device, smartphone, handheld computer, Smart Watch, medical device, point-of-care device.

15. A method of conducting Raman spectroscopy with a sensor module for Raman spectroscopy, the sensor module comprising a sensor package which encloses:
- an application specific integrated circuit (10), or ASIC,
- a light emitter arrangement (20) electrically connected to the ASIC (10),
- a light detector arrangement (30),
- a filter arrangement (40);
the method comprising the steps of:
- emitting light with multiple excitation wavelengths to excite Raman scattering in an external probe (60) to be placed outside of the sensor module,
- generating sensor signals from incident light emitted back from the external probe due to the Raman scattering,
- filtering the incident light according to a target passband, and
- using the ASIC, driving the light detector arrangement at the excitation wavelengths to shift a Raman spectral band of the external probe into the passband of the filter arrangement, wherein the sensor module is a sensor module for Raman spectroscopy according to one of claims 1 to 13.

## Patentansprüche

1. Sensormodul für Ramanspektroskopie, umfassend ein Sensorgehäuse, das Folgendes beinhaltet:
- eine anwendungsspezifische integrierte Schaltung (10), oder ASIC,
- eine Lichtemitteranordnung (20), die elektrisch mit dem ASIC (10) verbunden und so betreibbar ist, dass sie Licht mit mehreren Anregungswellenlängen emittiert, um eine Ramanstreuung in einer externen Sonde (60) anzuregen, die außerhalb des Sensormoduls anzuordnen ist,
- eine Lichtdetektoranordnung (30), die so betreibbar ist, dass sie Sensorsignale aus dem einfallenden Licht erzeugt, das aufgrund der Ramanstreuung von der externen Sonde (60) zurückemittiert wird, und
- eine Filteranordnung (40), die so betreibbar ist, dass sie das einfallende Licht entsprechend einem Ziel-Durchlassband (71) filtert; und wobei:
der ASIC (10) so betreibbar ist, dass er die Lichtemitteranordnung (20) bei den Anregungswellenlängen ansteuert, um ein Ramanspektralband der externen Sonde in das Durchlassband der Filteranordnung (40) zu verschieben, wobei der ASIC (10) ferner umfasst:
- einen Modulator, der so betreibbar ist, dass er ein AC-Ansteuersignal mit einer Modulationsfrequenz bereitstellt und ein dem AC-Ansteuersignal zugeordnetes Referenzsignal bereitstellt,
- einen Lock-in-Verstärker, der so betreibbar ist, dass er die Sensorsignale und das Referenzsignal vom Modulator empfängt und eine phasengesteuerte Detektion der Modulationsfrequenz in den Sensorsignalen durchführt, um unter Verwendung des Referenzsignals eine Phase und eine Amplitude aus den Sensorsignalen zu bestimmen.

2. Sensormodul nach Anspruch 1, wobei die Lichtemitteranordnung (20) einen einzelnen Lichtemitter (21) umfasst, der so betreibbar ist, dass er Licht mit mehreren Emissionslinien entsprechend den mehreren Anregungswellenlängen emittiert.

3. Sensormodul nach Anspruch 1 oder 2, wobei die Lichtemitteranordnung (20) einen einzelnen durchstimmbaren Lichtemitter (21) umfasst, der so betreibbar ist, dass er Licht mit durchstimmbaren Emissionslinien entsprechend den mehreren Anregungswellenlängen emittiert.

4. Sensormodul nach einem der Ansprüche 1 bis 3, wobei
- die Lichtemitteranordnung (20) eine Anordnung von Lichtemittern (21) mit unterschiedlichen Emissionslinien umfasst, und
- die Lichtemitter (21) der Anordnung so betreibbar sind, dass sie Licht mit mindestens einer Emissionslinie entsprechend den mehreren Anregungswellenlängen emittieren.

5. Sensormodul nach einem der Ansprüche 1 bis 4, wobei die Lichtemitteranordnung (20) als Lichtemitter zumindest einen von einer Laserdiode oder einem Oberflächenlaser, z.B. einem VCSEL, umfasst.

6. Sensormodul nach einem der Ansprüche 1 bis 5, wobei
- die Lichtdetektoranordnung (30) einen einzelnen Lichtdetektor (31) umfasst, und
- die Filteranordnung (40) einen Filter (41) umfasst, der vor dem Lichtdetektor (31) angeordnet ist.

7. Sensormodul nach einem der Ansprüche 1 bis 5, wobei
- die Lichtdetektoranordnung (30) eine Anordnung von Lichtdetektoren (31) umfasst, und
- die Filteranordnung (40) mehrere Filter (41) umfasst, die jeweils vor den Lichtdetektoren (31) angeordnet sind.

8. Sensormodul nach Anspruch 6 oder 7, wobei:
- der Filter (41) vor dem einzelnen Lichtdetektor (31) ein breites Durchlassband aufweist, oder ein Breitbandfilter, oder
- mehrere vor den Lichtdetektoren (31) angeordnete Filter schmale Durchbänder aufweisen, oder Schmalbandfilter.

9. Sensormodul nach Anspruch 8, wobei die Schmalbandfilter nicht überlappende Durchlassbänder mit diskreten Zentralwellenlängen aufweisen.

10. Sensormodul nach einem der Ansprüche 1 bis 9, wobei die Lichtdetektoranordnung (30) als Lichtdetektor (31) zumindest eines von einem Photonenzähler, z.B. eine Einzelphotonen-Lawinendiode, oder SPAD, einer Lawinenphotodiode, oder APD, einem Silizium-Photomultiplikator, oder SiPM, einer Fotodiode oder einem ladungsgekoppelten Bauelement, oder CCD, oder einem MEMS-Photomultiplikator, oder PM, umfasst.

11. Sensormodul nach einem der Ansprüche 1 bis 10, wobei
- das Sensorgehäuse ferner eine Linsenanordnung (50) beinhaltet, und
- die Linsenanordnung (50) so angeordnet ist, dass sie das emittierte Licht auf die externe Sonde (60) lenkt, um eine Ramanstreuung anzuregen, und/oder
- die Linsenanordnung (50) so angeordnet ist, dass sie das einfallende Licht auf die Lichtdetektoranordnung (30) lenkt.

12. Sensormodul nach Anspruch 11, wobei die Linsenanordnung (50) ferner so betreibbar ist, dass sie das emittierte Licht unter einem von der senkrechten Einfallsrichtung abweichenden Winkel auf die externe Sonde (60) lenkt, um eine schräge Beleuchtung zu erzeugen.

13. Sensormodul nach mindestens einem der vorigen Ansprüche, wobei die Lichtdetektoranordnung (30) in den ASIC (10) integriert ist und/oder die Halbleiter-Lichtemitteranordnung (20) in den ASIC (10) integriert ist.

14. Elektronisches Gerät, umfassend:
- ein Sensormodul für die Ramanspektroskopie gemäß einem der Ansprüche 1 bis 13, und
- ein Host-System, das eines von einem mobilen Gerät, einem Smartphone, einem Handheld-Computer, einer Smartwatch, einem medizinischen Gerät oder einem Point-of-Care-Gerät umfasst.

15. Verfahren zur Durchführung einer Ramanspektroskopie mit einem Sensormodul für die Ramanspektroskopie, wobei das Sensormodul ein Sensorgehäuse umfasst, das beinhaltet:
- eine anwendungsspezifische integrierte Schaltung (10), oder ASIC,
- eine Lichtemitteranordnung (20), die elektrisch mit dem ASIC (10) verbunden ist,
- eine Lichtdetektoranordnung (30),
- eine Filteranordnung (40);
wobei das Verfahren die folgenden Schritte umfasst:
- Emittieren von Licht mit mehreren Anregungswellenlängen, um eine Ramanstreuung in einer externen Sonde (60) anzuregen, die außerhalb des Sensormoduls anzuordnen ist,
- Erzeugen von Sensorsignalen aus dem einfallenden Licht, das aufgrund der Ramanstreuung von der externen Sonde zurückemittiert wird,
- Filtern des einfallenden Lichts gemäß einem Ziel-Durchlassband, und
- Ansteuern der Lichtdetektoranordnung bei den Anregungswellenlängen unter Verwendung des ASICs, um ein Ramanspektralband der externen Sonde in das Durchlassband der Filteranordnung zu verschieben, wobei das Sensormodul ein Sensormodul für die Ramanspektroskopie gemäß einem der Ansprüche 1 bis 13 ist.

## Revendications

1. Module de capteur pour la spectroscopie Raman, comprenant un boîtier de capteur qui renferme:
- un circuit intégré spécifique à une application (10), ou ASIC,
- un ensemble d'émetteurs de lumière (20) connecté électriquement à l'ASIC (10) et opérable pur émettre de la lumière à plusieurs longueurs d'onde d'excitation pour exciter une diffusion Raman dans une sonde externe (60) destinée à être placée à l'extérieur du module de capteur,
- un ensemble de détecteurs de lumière (30) opérable pour générer des signaux de capteur à partir de la lumière incidente réémise par la sonde externe (60) en raison de la diffusion Raman, et
- un ensemble de filtrage (40) opérable pour filtrer la lumière incidente selon une bande passante cible (71); et dans lequel:
l'ASIC (10) est opérable pour commander le ensemble d'émetteurs de lumière (20) aux longueurs d'onde d'excitation pour décaler une bande spectrale Raman de la sonde externe dans la bande passante du ensemble de filtrage (40), dans lequel l'ASIC (10) comprend en outre:
- un modulateur opérable pour fournir un signal de commande en courant alternatif ayant une fréquence de modulation et pour fournir un signal de référence associé au signal de commande en courant alternatif,
- un amplificateur à verrouillage de phase opérable pour recevoir les signaux du capteur et pour recevoir le signal de référence provenant du modulateur, et pour effectuer une détection à verrouillage de phase de la fréquence de modulation dans les signaux du capteur pour déterminer une phase et une amplitude à partir des signaux du capteur à l'aide du signal de référence.

2. Module de capteurs selon la revendication 1, dans lequel l'ensemble d'émetteurs de lumière (20) comprend un seul émetteur de lumière (21) opérable pour émettre de la lumière avec plusieurs raies d'émission correspondant aux multiples longueurs d'onde d'excitation.

3. Module de capteur selon la revendication 1 ou 2, dans lequel l'ensemble d'émetteurs de lumière (20) comprend un seul émetteur de lumière accordable (21) opérable pour émettre de la lumière avec des raies d'émission accordables correspondant aux multiples longueurs d'onde d'excitation.

4. Module de capteur selon l'une des revendications 1 à 3, dans lequel
- l'ensemble d'émetteurs de lumière (20) comprend un réseau d'émetteurs de lumière (21) avec les raies d'émission différentes, et
- les émetteurs de lumière (21) du réseau sont opérable pour émettre de la lumière avec au moins une raie d'émission correspondant aux multiples longueurs d'onde d'excitation.

5. Module de capteur selon l'une des revendications 1 à 4, dans lequel l'ensemble d'émetteurs de lumière (20) comprend au moins une diode laser ou un émetteur laser à surface d'émission, par exemple un VCSEL, en tant qu'émetteur de lumière.

6. Module de capteur selon l'une des revendications 1 à 5, dans lequel
- l'ensemble de détecteurs de lumière (30) comprend un seul détecteur de lumière (31), et
- l'ensemble de filtrage (40) comprend un filtre (41) qui est disposé en amont du détecteur de lumière (31).

7. Module de capteur selon l'une des revendications 1 à 5, dans lequel
- l'ensemble de détecteurs de lumière (30) comprend un réseau de détecteurs de lumière (31), et
- l'ensemble de filtrage (40) comprend plusieurs filtres (41) disposés respectivement devant les détecteurs de lumière (31).

8. Module de capteur selon la revendication 6 ou 7, dans lequel:
- le filtre (41) situé devant le seul détecteur de lumière (31) comprend une large bande passante, ou filtre à large bande, ou
- plusieurs filtres disposés devant les détecteurs de lumière (31) comprenant des bandes passantes étroites, ou filtres à bande étroite.

9. Module de capteur selon la revendication 8, dans lequel les filtres à bande étroite ont des bandes passantes qui ne se chevauchent pas, avec des longueurs d'onde centrales distinctes.

10. Module de capteur selon l'une des revendications 1 à 9, dans lequel l'ensemble de détecteurs de lumière (30) comprend au moins l'un des un compteur de photons, par exemple une diode à avalanche à photon unique, ou SPAD, une photodiode à avalanche, ou APD, un photomultiplicateur au silicium, ou SiPM, une photodiode ou un dispositif à couplage de charge, ou CCD, ou un photomultiplicateur MEMS, ou PM, en tant que détecteur de lumière (31).

11. Module de capteur selon l'une des revendications 1 à 10, dans lequel
- le boîtier du capteur renferme en outre un ensemble de lentilles (50), et
- l'ensemble de lentilles (50) est disposé pour diriger la lumière émise vers la sonde externe (60) pour exciter une diffusion Raman, et/ou
- l'ensemble de lentilles (50) est disposé pour diriger la lumière incidente vers l'ensemble de détection de lumière (30).

12. Module de capteur selon la revendication 11, dans lequel l'ensemble de lentilles (50) est en outre opérable pour diriger la lumière émise vers la sonde externe (60) sous un angle différent de l'incidence normale pour fournir un éclairage oblique.

13. Module de capteur selon au moins l'une des revendications précédentes, dans lequel l'ensemble de détecteurs de lumière (30) est intégré dans l'ASIC (10) et/ou l'ensemble d'émetteurs de lumière à semi-conducteur (20) est intégré dans l'ASIC (10).

14. Appareil électronique, comprenant:
- un module de capteur pour la spectroscopie Raman selon l'une des revendications 1 à 13, et
- un système hôte comprenant l'un des un appareil mobile, un smartphone, un ordinateur de poche, une montre connectée, un appareil médical, un appareil de diagnostic au chevet du patient.

15. Procédé de réaliser une spectroscopie Raman avec un module de capteur pour la spectroscopie Raman, le module de capteur comprenant un boîtier de capteur qui renferme:
- un circuit intégré spécifique à une application (10), ou ASIC,
- un ensemble d'émetteurs de lumière (20) connecté électriquement à l'ASIC (10),
- un ensemble de détecteurs de lumière (30),
- un ensemble de filtrage (40);
le procédé comprenant les étapes:
- émettre de lumière à plusieurs longueurs d'onde d'excitation pour exciter une diffusion Raman dans une sonde externe (60) destinée à être placée à l'extérieur du module de capteur,
- générer des signaux de capteur à partir de la lumière incidente réémise par la sonde externe en raison de la diffusion Raman,
- filtrer la lumière incidente selon une bande passante cible, et
- à l'aide du circuit ASIC, commander l'ensemble d'émetteurs de lumière aux longueurs d'onde d'excitation pour décaler une bande spectrale Raman de la sonde externe dans la bande passante du ensemble de filtrage, dans lequel le module de capteur est un module de capteur pour la spectroscopie Raman selon l'une des revendications 1 à 13.
